(19) Europäisches Patentamt
European Patent Office
Office européen des brevets

(11) Publication number : 0 576 227 A2

(12) # EUROPEAN PATENT APPLICATION

(21) Application number : 93304815.9

(51) Int. Cl.$^{5}$ : **C07H 19/04,** A61K 31/70

(22) Date of filing : 21.06.93

(30) Priority : 22.06.92 US 902304

(43) Date of publication of application :
29.12.93 Bulletin 93/52

(84) Designated Contracting States :
AT BE CH DE DK ES FR GB GR IE IT LI LU NL PT SE

(71) Applicant : ELI LILLY AND COMPANY
Lilly Corporate Center
Indianapolis Indiana 46285 (US)

(72) Inventor : Grindley, Gerald Burr
5223 East 77th Street
Indianapolis, Indiana 46250 (US)
Inventor : Grossman, Cora Sue
5838 Baron Court
Indianapolis, Indiana 46250 (US)
Inventor : Hertel, Larry Wayne
2313 Quiet Court
Indianapolis, Indiana 46239 (US)
Inventor : Kroin, Julian Stanley
8418 Hilltop Drive
Indianapolis, Indiana 46234 (US)

(74) Representative : Tapping, Kenneth George et al
Erl Wood Manor
Windlesham Surrey, GU20 6PH (GB)

(54) **2'-Deoxy-2',2'-difluoro(2,6,8-substituted) purine nucleosides having anti-viral and anti-cancer activity and intermediates.**

(57) 2-Deoxy-2,2-Difluoro (2,6,8-Substituted) Purine Nucleosides Having Anti-Cancer and Anti-Viral Activity and Intermediates.

EP 0 576 227 A2

This invention relates to substituted purine nucleosides having anti-tumor and anti-viral activity and methods for using same.

While the treatment of cancer was once considered impossible, great strides have been made to control the ravages of this often fatal disease. Several drugs which contribute to the increasing rate of survival are now routinely used clinically. The most commonly employed anti-tumor agents include methotrexate, doxorubicin and the vinca alkaloids such as, vincristine and purine nucleosides. Deoxydifluoro purine nucleosides that exhibit anti-tumor activity have been described by Hertel, et al., in Nucleotides and Nucleosides, 8, 951-55 (1989); and European Patent Specification Nos. 329,348; 339,161; 184,365; and 211,354.

It also known that antiviral agents are found among the general family of nucleosides. For example, 9-(2-hydroxyethoxymethyl)guanine is a potent antiviral agent against the herpes virus while deoxydifluoro purine nucleosides have been shown to exhibit potent anti-viral activity; see European Patent Specification Nos. 345,751; 122,707; and 339,161; and U.S. Patent Nos. 4,526,988; 4,965,374; and 4,692,434.

However, research continues to develop effective compounds with greater safety for the subjects under treatment.

According to the present invention there is provided a compound of the formula

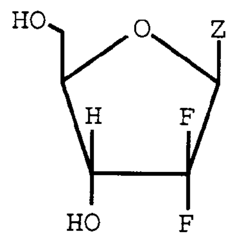

(I);

wherein Z is selected from the group consisting of

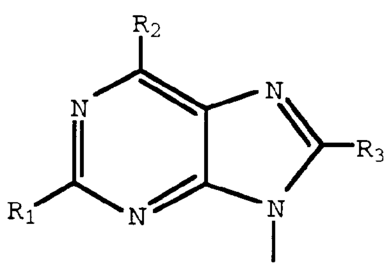

,

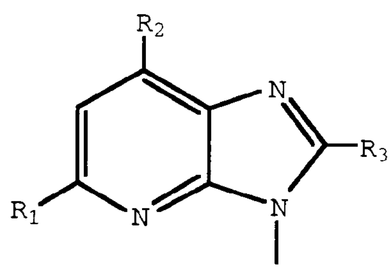

,

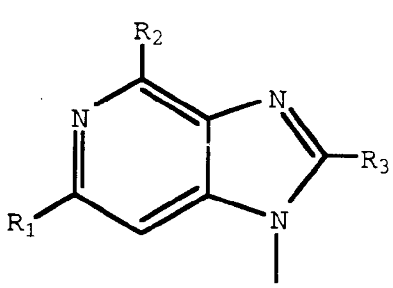

,

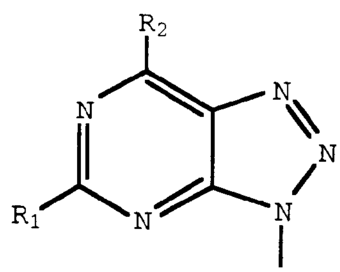

,

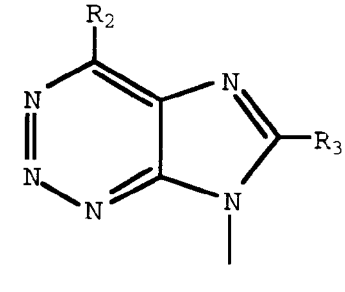

and

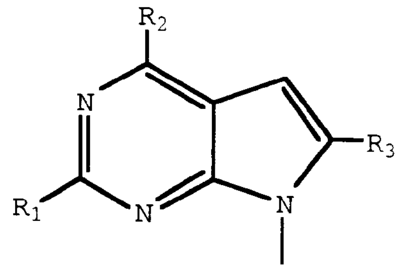

;

wherein $R_1$ and $R_2$ are independently selected from the group consisting of alkyl, alkylhalo alkoxy, amine, halo, hydrogen, hydroxy, cyano, thio, thioalkyl, hydrazide, carboxamide, thioamide, sulfonamide, sulfinamide, alkylamine, thioamine, hydroxyamine, NH(alkyl), N(alkyl)$_2$, O(aryl), O(substituted aryl), N(aryl), N(substituted aryl); and $R_3$ is selected from the group consisting of hydrogen, hydroxy, amine, NH(alkyl), halo, alkoxy, thioalkyl; and pharmaceutically acceptable salts thereof.

The term "alkyl" alone or in combination refers to straight, cyclic or branched chain aliphatic hydrocarbon groups containing 1 to 7 carbon atoms and more preferably containing 1 to 4 carbon atoms such as, methyl, ethyl, n-propyl, isopropyl, n-butyl, t-butyl, isobutyl and sec-butyl. The term "aryl" alone or in combination refers to carbocyclic or heterocyclic groups such as phenyl, naphthyl, thienyl and substituted derivatives thereof. The term "aromatic" alone or in combination refers to benzene-like structures containing (4n+2) $\pi$ delocalized electrons. The term "substituted" alone or in combination refers to substitution by one or more of the groups selected from cyano, halo, carboalkoxy, toluoyl, nitro, alkoxy, alkyl, amino and dialkylamino. The term "halo" alone or in combination refers to chloro, fluoro, bromo or iodo. The term "alkoxy" alone or in combination refers to the general formula AO; wherein A is alkyl. The term "thioalkyl" alone or in combination refers to the general formula BS; wherein B is alkyl or hydrogen. The phrase "anti-virally effective amount" refers to an appropriate amount of a compound of formula (I) which is capable of preventing or inhibiting the presence of viral infections in mammals. The phrase "susceptible neoplasm" refers an abnormal growth of tissue in mammals capable of being treated by a compound of formula (I). The phrase "pharmaceutically effective amount" refers to an amount of a compound of formula (I) capable of treating mammals suffering from disease states stemming from tumors and viral infections. The phrase "inert solvent" refers to substances that provide a medium in which a reaction can occur but otherwise do not materially contribute to the reaction. The phrase "active ingredient" alone or in combination refers to a compound of formula (I) or a pharmaceutically acceptable salt thereof. The phrase "pharmaceutically acceptable" refers to a carrier, diluent or excipient compatible with the other ingredients of the formulation and not deleterious to the recipient thereof. The phrase "unit dosage form" refers to physically discrete units suitable as unitary dosages for human subjects and other mammals, each unit containing a predetermined quantity of active material calculated to produce the desired therapeutic effect, in association with a suitable pharmaceutical carrier.

In one aspect, the invention provides intermediate compounds of formula (II) useful in the preparation of compounds of formula (I).

The intermediate compounds of formula (II) are prepared by coupling a 2-deoxy-2,2-difluorocarbohydrate, as described in U.S. Patent 4,526,988, having a leaving group at C-1, with a nucleobase. The leaving groups placed on the 2-deoxy-2,2-difluorocarbohydrate, to facilitate coupling, are those typically used in organic synthesis and may be selected from toluenesulfonate, ethanesulfonate, isopropanesulfonate, p-methoxybenzenesulfonate, p-nitrobenzenesulfonate, m-chlorobenzenesulfonate and halo; preferred is halo; while most preferred is iodo.

Halo leaving groups are readily provided by reacting a 2-deoxy-2,2-difluorocarbohydrate with an arylsulfonyl halide or arylsulfonyl anhydride in the presence of an equivalent amount of a suitable acid scavenger such as triethylamine. This results in an arylsulfonyl leaving group being placed at C-1. The arylsulfonyl leaving group is then displaced by a halo leaving group with, for example, tetrabutylammoniumiodide.

It is generally desirable to convert the free hydroxy groups of the 2-deoxy-2,2-difluorocarbohydrate to protected hydroxy groups (X) to prevent them from reacting with the nucleobase. The protecting groups are those commonly used in synthetic organic chemistry. Chemists are accustomed to choosing protecting groups which can be efficiently placed on the 2-deoxy-2,2-difluorocarbohydrate and easily removed therefrom once the reaction is complete. Hydroxy protecting groups known in the art are described in Chapter 3 of Protective Groups in Organic Chemistry, McOmie Ed., Plenum Press, New York (1973), and Chapter 2 of Protective Groups in Organic Synthesis, Green, John, J. Wiley and Sons, New York (1981); preferred are benzoyl, mono-substituted

benzoyl and disubstituted benzoyl, acetyl, pivalamido, triphenylmethyl ethers, and silyl ether forming groups, especially t-butyldimethylsilyl; while most preferred is benzoyl.

In attaching the hydroxy protecting groups to the 2-deoxy-2,2-difluorocarbohydrate, typical reaction conditions are employed depending on the nature of the protecting group chosen and are discussed in U.S. Patent 4,526,988, which is incorporated herein by reference.

The nucleobase materials used in forming the compounds of formula (I) are known to those of ordinary skill in the art, therefore no discussion of their synthesis is necessary. The amino groups present on some of the nucleobases, however, may be protected before the nucleobase is coupled with the 2-deoxy-2,2-difluorocarbohydrate. The amino-protecting groups employed are preferably selected from silyl, t-butoxycarbonyl, benzyloxycarbonyl, 4-methoxybenzyloxycarbonyl, 4-nitrobenzyloxycarbonyl, formyl, and acetyl.

It is advisable to convert the keto oxygen atoms on the nucleobase to the enol form, in order to make them more aromatic and allow for ready attachment of the nucleobase and 2-deoxy-2,2-difluorocarbohydrate. Enolization is provided most conveniently by producing the silyl protecting groups. The silyl protecting groups, discussed above, may be used for this purpose.

The coupling reaction between the 2-deoxy-2,2-difluorocarbohydrate and the nucleobase is carried out at about 50°C to about 200°C and preferably in a solvent such as dimethylformamide, dimethylacetamide or hexamethylphosphoramide. If the coupling reaction is carried out at elevated pressures, to avoid the distillation of low-boiling solvents, any convenient inert reaction solvent may be used.

An alternative process for preparing intermediate compounds of formula (II) is described by Hertel in, Synthesis, Cytotoxicity and Metabolism of the 2',2'-Difluoro-Analogs of Deoxyadenosine (dFdA) and Deoxyguanosine (dFdG), Nucleosides & Nucleotides, 8(5&6), p 951-55 (1989).

The C-2, C-6 and C-8 substituent positions of the purine nucleobase may be modified by procedures known to those of ordinary skill in the art to form the intermediates of formula (II) which may be deblocked to form the compounds of formula (I). For example, a halogen at one or more of these positions may be displaced by nucleophilic substitution and the resulting nucleophile substituent modified to produce the desired substituent. A cyano nucleophile could, for example, be oxidized to form a carboxamide or reduced to form a methyleneamine.

To obtain a compound of formula (I) requires the removal of the protecting groups.

Most silyl protecting groups are cleaved easily with water or alcohol. The removal of t-butyldimethylsilyl protecting group requires acid conditions, such as contact with gaseous hydrogen halide.

Acyl protecting groups are removed by simple hydrolysis with strong or moderately strong base, such as alkali metal hydroxides, at temperatures from about ambient temperature to about 100°C. At least one equivalent amount of base is needed for each protecting group. Such hydrolysis is conveniently carried out in hydroxylic solvents, especially aqueous alcohols. The reactions also may be carried out, however, in any convenient solvent such as polyols including ethylene glycol, ethers such as tetrahydrofuran, ketones such as acetone and methyl ethyl ketone and other polar solvents such as dimethylsulfoxide.

The cleavage of acyl protecting groups may be performed with other bases, including, for example, sodium methoxide, potassium t-butoxide, hydrazine, hydroxylamine, ammonia, alkali metal amides and secondary amines such as diethylamine.

The acyl protecting groups also can be removed with acid catalysts, such as methanesulfonic acid, hydrochloric acid, hydrobromic acid, sulfuric acid, or with acidic ion exchange resins.

Preferably the hydrolysis is carried out at a relatively high temperatures, such as the reflux temperature of the mixture, but temperatures as low as ambient may be used when particularly strong acids are employed.

The removal of ether protecting groups is carried out by known methods, for example, with ethanethiol and aluminum chloride.

Compounds of the invention that possess hydroxy or amino acyl or alkyl groups can, of course, be either selectively deprotected, or such groups may be removed and selectively replaced by standard conditions.

None of the reaction steps require unusual excesses of the reactants. As is common in organic syntheses, use of a moderate excess or reagents, in the range of 1.05X to 2X, is advisable.

Modifications to the above processes may be necessary to accommodate the reactive functionalities of particular substitutents. Such modifications would be apparent to those of ordinary skill in the art.

The following illustrate compounds of formula (I) contemplated within the scope of the present invention:

(a) 2-deoxy-2,2-difluoro-D-ribofuranosyl-1-β-(2-amino-6-chloropurine);
(b) 2-deoxy-2,2-difluoro-D-ribofuranosyl-1-β-(2-amino-6 methoxypurine);
(c) 2-deoxy-2,2-difluoro-D-ribofuranosyl-1-β-(2-amino-6-ethoxypurine);
(d) 2-deoxy-2,2-difluoro-D-ribofuranosyl-1-β-(2-amino-6-dimethylaminopurine);
(e) 2-deoxy-2,2-difluoro-D-ribofuranosyl-1-β-(2-amino-6-methylaminopurine);
(f) 2-deoxy-2,2-difluoro-D-ribofuranosyl-1-β-(2-amino-6-iodopurine);

(g) 2-deoxy-2,2-difluoro-D-ribofuranosyl-1-β-(2-amino-6-cyanopurine);
(h) 2-deoxy-2,2-difluoro-D-ribofuranosyl-1-β-(2-amino-6-thiopurine);
(i) 2-deoxy-2,2-difluoro-D-ribofuranosyl-1-β-(2-amino-6-carboxamidopurine);
(j) 2-deoxy-2,2-difluoro-D-ribofuranosyl-1-β-(2-hydroxyadenosine);
(k) 2-deoxy-2,2-difluoro-D-ribofuranosyl-1-β-(2-fluoroadenosine);
(l) 2-deoxy-2,2-difluoro-D-ribofuranosyl-1-β-purine;
(m) 2-deoxy-2,2-difluoro-D-ribofuranosyl-1-β-(2-aminopurine);
(n) 2-deoxy-2,2-difluoro-D-ribofuranosyl-1-β-(2-amino-6-methylpurine);
(o) 2-deoxy-2,2-difluoro-D-ribofuranosyl-1-β-(2-amino-6-chloro-1-deazapurine);
(p) 2-deoxy-2,2-difluoro-D-ribofuranosyl-1-β-(6-chloro-7-deazapurine);
(q) 2-deoxy-2,2-difluoro-D-ribofuranosyl-1-β-(2,6-dichloro-3-deazapurine);
(r) 2-deoxy-2,2-difluoro-D-ribofuranosyl-1-β-(6-amino-3-deazapurine);
(s) 2-deoxy-2,2-difluoro-D-ribofuranosyl-1-β-(2,6-chloropurine);

and pharmaceutically acceptable salts thereof.

Preferred compounds of formula (I) are compounds (a) thru (f) and pharmaceutically acceptable salts thereof.

Although generally neutral, particular compounds of formula (I) can possess sufficiently acidic or basic functional groups to react with any of a number of inorganic bases and inorganic and organic acids, to form a pharmaceutically acceptable salt. Acids commonly employed to form acid addition salts are inorganic acids such as hydrochloric acid, hydrobromic acid, hydroiodic acid, sulfuric acid, phosphoric acid, sodium, potassium, lithium or ammonium acid addition salts and the like, and organic acids such as p-toluenesulfonic acid, methanesulfonic acid, oxalic acid, p-bromophenylsulfonic acid, carbonic acid, succinic acid, citric acid, benzoic acid, acetic acid, and the like. Examples of such pharmaceutically acceptable salts thus are the sulfate, pyrosulfate, bisulfate, sulfite, bisulfite, phosphate, monohydrogenphosphate, chloride, bromide, iodide, acetate, propionate, decanoate, caprylate, acrylate, formate, isobutyrate, caproate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, sulfonate, xylenesulfonate, phenylacetate, phenylpropionate, phenylbutyrate, citrate, lactate, hydroxybutyrate, glycollate, tartrate, methanesulfonate, propanesulfonate, naphthalene-l-sulfonate, naphthalene-2-sulfonate, mandelate, and the like. Preferred pharmaceutically acceptable acid addition salts are those formed with mineral acids such as hydrochloric acid and hydrobromic acid, and those formed with organic acids such as maleic acid and methanesulfonic acid.

Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, sodium carbonate, sodium bicarbonate, potassium bicarbonate, calcium hydroxide, calcium carbonate, and the like. The potassium and sodium salt forms are particularly preferred.

It is recognized that various anomeric forms of the compound of formulas (I) and (II) may exist. However, this invention is not limited to any particular anomer but rather includes all single anomers and mixtures thereof; preferred are the beta-anomers.

The compound of formula (I) and pharmaceutically acceptable salts thereof can also exist as various solvates, such as with water, methanol, ethanol, dimethylformamide, and the like. Mixtures of such solvates can also be prepared. The source of such solvate can be from the solvent of crystallization, inherent in the solvent of preparation or crystallization, or adventitious to such solvent. These solvates are also within the scope of the present invention.

The pharmaceutically acceptable salts embraced by formula (I) of the present invention are prepared by reacting an equimolar or excess amount of an acid or base with a compound of formula (I) in a suitable mutual inert or substantially inert solvent or a mixture of solvents. The particular choice of solvent will depend on the relative solubility of the starting materials and resultant salts, and slurries rather than solutions of certain reagents may be used to obtain the salts. The salt forming reaction is carried out at about -10°C to about 100°C, preferably about room temperature and the solvent is removed by conventional means.

The following examples illustrate specific aspects of the present invention and are not intended to limit the scope thereof in any respect and should not be so construed.

## Example 1

2-deoxy-2,2-difluoro-D-ribofuranosyl-1-β-(2-amino-6-chloropurine)

To a suspension of 2-amino-6-chloropurine (307 mmol, 52 g) in dimethylacetamide (2 L) at 0°C under nitrogen was added powdered potassium hydroxide (368 mmol, 20.6 g). The mixture was stirred for 30 minutes to form a solution. 2-deoxy-2,2-difluoro-D-ribofuranosyl-3,5-dibenzoyl-1-α-iodide (307 mmol, 150 g) in dimethylacetamide (400 ml) was added. The reaction mixture was allowed to warm to room temperature and stirred under nitrogen. The reaction was then quenched with brine and extracted with ethyl acetate. The organic layer was washed successively with brine, 1N HCl, saturated sodium bicarbonate solution, and water, then dried over sodium sulfate and evaporated in vacuo to provide the crude product.

The crude product was purified with silica gel chromatography to yield a 3:1 beta to alpha anomer ratio of of 2-deoxy-2,2-difluoro-D-ribofuranosyl-3,5-dibenzoyl-1-(2-amino-6-chloropurine) intermediate. The beta anomer of the 2-deoxy-2,2-difluoro-D-ribofuranosyl-3,5-dibenzoyl-1-(2-amino-6-chloropurine) intermediate (47.22 g) was isolated by crystallization in an isolated yield of 24 percent.

The intermediate was deprotected by suspending it in methanol at 0°C and saturating the mixture with anhydrous ammonia. The resulting solution was warmed to room temperature and stirred overnight. The solution was then purged with nitrogen and evaporated to obtain the titled product which was then purified by washing with a non-polar solvent such as methylene chloride to remove the benzoate byproducts. $^1$H NMR (300 MHz, $CD_3OD$), δ 3.90 (m, 3H, 4'-H,5'-H), 4.58 (m, 1H, 3'-H), 6.27 (dd, 1H, 1'-H), 8.31 (s, 1H, 8-H).

Example 2

2-deoxy-2,2-difluoro-D-ribofuranosyl-1-β-(2-amino-6-methoxypurine)

The 3:1 beta to alpha anomer ratio of the product from Example 1 (0.5 mmol, 160.5 mg) was mixed with methanol (25 ml) and sodium methoxide (1 mmol, 54 mg). The solution was then refluxed under nitrogen for 2 hours, quenched with water. The titled product (75.3 mg) was evaporated to an oil and separated by reversed phase HPLC in an isolated yield of 48 percent. FD-MS m/e 317=M; $^1$H NMR (300 MHz, $CD_3OD$), δ 3.88 (m, 3H, 4'-H,5'-H), 4.03 (s, 3H, OMe), 4.58 (m, 1H, 3'-H), 6.17 (dd, 1H, 1'-H), 8.08 (s, 1H, 8-H).

Example 3

2-deoxy-2,2-difluoro-D-ribofuranosyl-1-β-(2-amino-6-ethoxypurine)

The product from Example 1 (3.11 mmol, 1.0 g) was added to ethanol (100 ml) and sodium ethoxide (6.22 mmol, 423 mg). The solution was refluxed under nitrogen for 2 hours, quenched with water to provide the titled product and evaporated to a solid. The titled product (805 mg) was isolated by silica gel chromatography in an isolated yield of 78 percent. FD-MS m/e 331=M; Anal. $C_{12}H_{15}F_2N_5O_4$ Calc.: C, 43.51; H, 4.56; N, 21.14. Actual: C, 43.40; H, 4.62; N, 20.88 $^1$H NMR (300 MHz, $CD_3OD$), δ 1.42 (t, 3H, 6-$OCH_2\underline{CH_3}$), 3.89 (m, 3H, 4'-H,5'-H), 4.54 (m, 3H, 3'-H,6-$O\underline{CH_2}CH_3$), 6.18 (dd, 1H, 1'-H), 8.06 (s, 1H, 8- H).

Example 4

2-deoxy-2,2-difluoro-D-ribofuranosyl-1-β-(2-amino-6-dimethylaminopurine)

The product from Example 1 (.5 mmol, 160.5 mg) was added to methanol (25 ml), dimethylamine (2.5 ml of a 40 percent solution in water), and stirred at room temperature for 2 hours. The titled product (165 mg) was isolated by evaporating the solvents in vacuo in an isolated yield of 100 percent. FD-MS m/e 330=M; $^1$H NMR (300 MHz, $CD_3OD$), δ 2.66 (s, 6H, 6-N(CH3)2), 3.86 (m, 3H, 4'-H,5'-H), 4.53 (m, 1H, 3'-H), 6.13 (dd, 1H, 1'-H), 7.91 (s, 1H, 8-H).

Example 5

2-deoxy-2,2-difluoro-D-ribofuranosyl-1-β-(2-amino-6-methylaminopurine)

The product from Example 1 (0.2 mmol, 64.3 mg) was added to ethanol (10 ml), methylamine (1 ml of a 40% solution in water), and stirred at room temperature for 2 1/2 hours. The titled product (48.7 mg) was obtained by evaporating the solvents in vacuo, and isolated by silica gel chromatography in a yield of 77 percent. FD-MS m/e 316=M; $^1$H NMR (300 MHz, $CD_3OD$), δ 3.03 (s, 3H, 6-$NH\underline{CH_3}$), 3.88 (m, 3H, 4'-H,5'-H), 4.58(m, 1H, 3'-H), 6.12(dd, 1H, 1'-H), 7.93 (s, 1H, 8-H).

Example 6

2-deoxy-2,2-difluoro-D-ribofuranosyl-1-β-(2-amino-6-iodopurine)

The product from Example 1 (3.1 mmol, 1.0 g) was added to hydriodic acid (10 ml, 47 to 51 percent) at 10°C. The resulting solution was stirred at this temperature for 2 hours. The solution was diluted with ice and

neutralized with cold concentrated ammonium hydroxide and evaporated. The mixture was partitioned between ethyl acetate and water. The titled product (1.05 g) was collected from the organic layer by drying over sodium sulfate and evaporating in vacuo in an isolated yield of 82 percent. FD-MS m/e 413=M; $^1$H NMR (300 MHz, $CD_3OD$), δ 3.90 (m, 3H, 4'-H,5'-H), 4.57 (m, 1H, 3'-H), 6.17 (dd, 1H, 1'-H), 8.30 (s, 1H, 8-H).

Example 7

2-deoxy-2,2-difluoro-D-ribofuranosyl-1-β-(2-amino-6-cyanopurine)

To a mixture of cuprous cyanide (1 mmol, 89.5 mg) in pyridine (5 ml) was added the product from Example 6 (0.25 mmol, 164 mg of 63 percent pure). The mixture was heated at 130°C-135°C for 10 minutes and evaporated to dryness. The resulting black residue was extracted with hot acetonitrile (3 x 5 ml). The combined extracts were evaporated in vacuo and the resulting residue was extracted with hot isopropanol (3 x 5 ml). The combined extract was evaporated in vacuo to a brown solid. The titled product (20 mg) was isolated by silica gel chromatography in an isolated yield of 26 percent. FD-MS m/e 312=M; $^1$H NMR (300 MHz, $CD_3OD$), δ 3.90 (m, 3H, 4'-H,5'-H), 4.57(m, 1H, 3'-H), 6.27 (dd, 1H, 1'-H), 8.48 (s, 1H, 8-H).

Example 8

2-deoxy-2,2-difluoro-D-ribofuranosyl-1-β-(2-amino-6-carboxamidopurine)

The product from Example 7 (0.15 mmol, 47 mg) was added to methanol (2.5 ml) and water (2.5 ml) and cooled to -10°C. The pH of the solution was adjusted to 8.5 with concentrated ammonium hydroxide. Hydrogen peroxide (.24 mmol, 0.02 ml, 30 percent) was added and the reaction mixture was allowed to warm to room temperature. After 2 hours hydrogen peroxide (.02 ml) was added and the reaction was continued for another hour. The titled product was collected by evaporating the solvents. FD-MS m/e 331=M+1; $^1$H NMR (300 MHz, $CD_3OD$), δ 3.90 (m, 3H, 4'-H,5'-H), 4.57 (m, 1H, 3'-H), 6.28 (dd, 1H, 1'-H), 8.38 (s, 1H, 8-H).

Example 9

2-deoxy-2,2-difluoro-D-ribofuranosyl-1-β-(2-hydroxyadenosine)

A solution of 2-deoxy-2,2-difluoro-D-ribofuranosyl-1-(2,6-diaminopurine) (2 mmol, 604 mg), sodium nitrite (12 mmol, 828 mg) and water (15 ml) was warmed to 60°C and treated with glacial acetic acid (1 ml). After 5 minutes the solution was cooled to room temperature. The titled product was purified by passing the solution thru an HP20 resin and eluting with methanol, followed by water. The aqueous layer was evaporated in vacuo. FD-MS m/e 303=M; $^1$H NMR (300 MHz, $CD_3OD$), δ 3.70 (m, 3H, 4'-H,5'-H), 4.44 (m, 1H, 3'-H), 5.98 (dd, 1H, 1'-H), 7.86 (s, 1H, 8-H).

Example 10

2-deoxy-2,2-difluoro-D-ribofuranosyl-1-β-(2-fluoroadenosine)

To a solution of 2-deoxy-2,2-difluoro-D-ribofuranosyl-3,5-dibenzoyl-1-(2,6-diaminopurine) (2 mmol, 1.02 g) in a hydrogen fluoride-pyridine mixture (17 ml, 60:40 ratio) at -30°C was added tert-butylnitrite (2.8 mmol, 0.33 ml). The mixture was stirred for 30 minutes, then poured into ice-water and separated by extraction into methylene chloride. The organic layer was washed with water and saturated sodium bicarbonate solution, then evaporated in vacuo. Purification by silica gel chromatography yielded 2-deoxy-2,2-difluoro-D-ribofuranosyl-3,5-dibenzoyl-1-(2-fluoroadenosine) (500 mg; 49%). This intermediate (2 mmol, 1.03 g) was dissolved in tetrahydrofuran (50 ml) and treated with lithium aluminum hydride (1 mmol, 38 mg). The resulting mixture was stirred under nitrogen for 2 hours. Lithium aluminum hydride (4 x 1 mmol) was added to the mixture over 48 hours. The reaction was quenched by the dropwise addition of water, then poured into water (100 ml). The product was extracted into ethyl acetate, washed with brine, dried over sodium sulfate and evaporated in vacuo. Addition of methylene chloride gave the titled product (425 mg) as a solid, which was collected by filtration in an isolated yield of 70 percent. FD-MS m/e 305=M; FD-FAB m/e 306.08176=M+1; (Calc. 306.08137); $^1$H NMR (300 MHz, $CD_3OD$), δ 3.88 (m, 3H, 4'-H,5'-H), 4.56 (m, 1H, 3'-H), 6.18 (dd, 1H, 1'-H), 8.08 (s, 1H, 8-H).

By substantially following the procedure described in Example 1 and using the appropriate purine nucleobase, the compounds of Examples 11 thru 14 were prepared.

Example 11

2-deoxy-2,2-difluoro-D-ribofuranosyl-1-β-purine

FD-MS m/e 273=M+1; $^1H$ NMR (300 MHz, $CD_3OD$), δ 3.88(m, 3H, 4'-F, 5'-H), 4.57(m, 1H, 3'-H), 6.47(dd, 1H, 1'-H), 8.80(s, 1H, 8-H), 8.97(s, 1H, 2-H), 9.11(s, 1H, 6-H).

Example 12

2-deoxy-2,2-difluoro-D-ribofuranosyl-1-β-(2-aminopurine)
FD-MS m/e 287=M; $^1H$ NMR (300 MHz, $CD_3OD$), δ 3.84(m, 3H, 4'-F, 5'-H), 4.52(m, 1H, 3'-H), 6.22(dd, 1H, 1'-H), 8.30(s, 1H, 8-H), 8.97(s, 1H, 2-H), 8.56(s, 1H, 6-H).

Example 13

2-deoxy-2,2-difluoro-D-ribofuranosyl-1-β-(2-amino-6-methylpurine)
FD-MS m/e 301=M; $^1H$ NMR (300 MHz, $CD_3OD$), d2.05 (s, 3H, 6-Me), δ 3.88 (m, 3H, 4'-H,5'-H), 4.58 (m, 1H, 3'-H), 6.22 (dd, 1H, 1'-H), 8.23 (s, 1H, 8-H).

Example 14

2-deoxy-2,2-difluoro-D-ribofuranosyl-1-β-(6-chloro-7-deazapurine)
FD-MS m/e 514=M + 1;

Example 15

2-deoxy-2,2-difluoro-D-ribofuranosyl-1-β-(7-deaza adenosine)
The product from Example 14 (3.54 g, 6.89 mmol) was treated with saturated ammonia/methanol solution. The solution was heated to 80°C for 48 hours, quenched with water to provide the titled product and evaporated to a solid. 1.54 g of the solid was isolated by silica gel chromatography in a yield of 76 percent. MS m/e 287=M + 1; Anal. $C_{11}H_{12}F_2N_4O_3$·.05$H_2O$ Calc.: C, 44.75; H, 4.44; N, 18.98. Actual: C, 44.87; H, 4.50; N,18.86.

Example 16

2-deoxy-2,2-difluoro-D-ribofuranosyl-1-β-(2,6-chloropurine)
To a solution of 2,6-dichloropurine (15 mmol, 2.84 g) in 150 ml anhydrous tetrahydrofuran was added triphenylphosphine (18.8 mmol, 4.90 g) and diethylazodicarboxylate (18.8 mmol, 2.96 ml). 2-deoxy-2,2-difluoro-D-ribofuranosyl-3,5-dibenzoate (15 mmol, 5.67 g) was added as a solution in anhydrous tetrahydrofuran. The solution was stirred at room temperature under nitrogen overnight to form a 2-deoxy-2,2-difluoro-D-ribofuranosyl-3,5-dibenzoyl-1-β-(2,6-chloropurine) intermediate (3.78 g) in a yield of 46 percent. The intermediate was deblocked by adding diisopropylethylamine in methanol. The titled product was isolated by reversed phase HPLC. $^1H$ NMR (300 MHz, $CD_3OD$), δ 3.93 (m, 3H, 4'-H,5'-H), 4.60 (m, 1H, 3'-H), 6.34 (dd, 1H, 1'-H), 8.54 (s, 1H, 8-H).

The compounds of formula (I) can be administered by a variety of routes including oral, rectal, transdermal, subcutaneous, intravenous, intramusclar, and intranasal.

In another aspect, the invention provides pharmaceutical formulations containing a compound of formula (I) or a pharmaceutically acceptable salt thereof, in association with a pharmaceutically acceptable carrier, diluent or excipient. The compounds of formula (I) are preferably formulated prior to administration.

The active ingredient in such formulations comprises from 0.1 percent to 99.9 percent by weight of the formulation.

The pharmaceutical formulations are prepared by procedures well known and with readily available ingredients. In making the compositions, the active ingredient is preferably admixed with a carrier, or diluted by a carrier, or enclosed within a carrier which may be in the form of a capsule, sachet, paper or other container. When the carrier serves as a diluent, it may be a solid, semi-solid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosols, (as a solid or in a liquid medium), ointments containing, for example, up to 10 percent by weight of the active compound, soft and hard gelatin capsules, suppositories, sterile injectable solutions, sterile packaged powders, and the like.

Examples of suitable carriers, excipients, and diluents are lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, methyl cellulose, methylhydroxybenzoates, propylhydroxybenzoates, talc, magnesium stearate and mineral oil. The formulations may additionally include lubricating agents, wetting agents, sweetening agents, flavoring agents, emulsifying and suspending agents, preserving agents, and the like. The compositions of the invention may be formulated so as to provide quick, sustained or delayed release of the active ingredient after administration to the patient by employing procedures well known in the art.

The compositions are preferably formulated in a unit dosage or, each dosage generally containing from about 0.1 mg to about 3,000 mg, and preferably from about 1 mg to about 500 mg, of the active ingredient. However, it will be understood that the amount of compound actually administered will be determined by a physician, in the light of the relevant circumstances including the condition to be treated, the particular compound to be administered, the chosen route of administration, the age, weight, and response of the individual patient, and the severity of the patient's symptoms, and therefore the above dosage ranges are not intended to limit the scope of the invention in any way.

Formulation 1

Hard gelatin capsules are prepared using the following ingredients:

| | Quantity (mg/capsule) |
|---|---|
| Active ingredient | 250 |
| Starch, dried | 200 |
| Magnesium | 10 |
| Total | 460 mg |

The above ingredients are mixed and filled into hard gelatin capsules in 460 mg quantities.

Formulation 2

A tablet is prepared using the ingredients below:

| | Quantity (mg/capsule) |
|---|---|
| Active ingredient | 250 |
| Cellulose, microcrystalline | 400 |
| Silicon dioxide, fumed | 10 |
| Stearate acid | 5 |
| Total | 665 mg |

The components are blended and compressed to form tablets each weighing 665 mg.

Formulation 3

An aerosol solution is prepared containing the following components:

| | Weight Percent |
|---|---|
| Active ingredient | 0.25 |
| Ethanol | 29.75 |
| Propellant 22 (Chlorodifluoromethane) | 70.00 |

The active compound is mixed with ethanol and the mixture added to a portion of the propellant 22, collected to -30°C and transferred to a filling device. The required amount is then placed in a stainless steel container and diluted with the remainder of the propellant. The valve units are then fitted to the container.

Formulation 4

Tablets, each containing 60 mg of active ingredient, are made as follows:

| | |
|---|---|
| Active ingredient | 60 mg |
| Starch | 45 mg |
| Microcrystalline cellulose | 35 mg |
| Polyvinylpyrrolidonne (as 10% solution in water) | 4 mg |
| Sodium carboxymethyl starch | 4.5 mg |
| Magnesium stearate starch | 0.5 mg |
| Talc | 1 mg |
| Total | 150 mg |

The active ingredient, starch and cellulose are passed through a No. 45 mesh U.S. sieve and mixed thoroughly. The aqueous solution containing polyvinylpyrrolidone is mixed with the resultant powder, and the mixture then is passed through a No. 14 mesh U.S. sieve. The granules so produced are dried at 50°C and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

Formulation 5

Capsules, each containing 80 mg of active ingredient, are made as follows:

| | |
|---|---|
| Active ingredient | 80 mg |
| Starch | 59 mg |
| Microcrystalline cellulose | 59 mg |
| Magnesium stearate | 2 mg |
| Total | 200 mg |

The active ingredient, cellulose, starch, and magnesium stearate are blended, passed through a No. 45 mesh U.S. sieve, and filled into hard gelatin capsules in 200 mg quantities.

Formulation 6

Suppositories, each containing 225 mg of active ingredient, are made as follows:

| | |
|---|---|
| Active ingredient | 225 mg |
| Saturated fatty acid glycerides | 2,000 mg |
| Total | 2,225 mg |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

Formulation 7

Suspensions, such containing 50 mg of active ingredient per 5 ml dose, are made as follows:

| | |
|---|---|
| Active ingredient | 50 mg |
| Sodium carboxymethyl cellulose | 50 mg |
| Syrup | 1.25 ml |
| Benzoic acid solution | 0.10 ml |
| Flavor | q.v. |
| Color | q.v. |
| Purified water | 5 ml |

The active ingredient is passed through a No. 45 mesh U.S. sieve and mixed with the sodium carboxymethyl cellulose and syrup to form a smooth paste. The benzoic acid solution, flavor and color are diluted with a portion of the water and added, with stirring. Sufficient water is then added to produce the required volume.

Formulation 8

An intravenous formulation may be prepared as follows:

| | |
|---|---|
| Active ingredient | 100 mg |
| Sodium alginate | 500 mg |
| Isotonic saline | 1,000 ml |

The solution of the above ingredients is generally administered intravenously to a subject at a rate of 1 ml per minute.

In another aspect, the invention provides a method of treating susceptible neoplasms in mammals comprising administering to a mammal in need of such treatment a pharmaceutically effective amount of a compound of formula (I).

The compounds of formula (I) and their pharmaceutically acceptable salts are surprisingly advantageous for treating mammals suffering from disease states stemming from tumors. The treatment contemplated by the present method includes both medical therapeutic and prophylactic treatment, as appropriate. The specific dosage level of the compound administered according to this invention will, of course, be determined by the circumstances surrounding the case, including, for example, the particular compound administered, the route of administration, and the condition being treated. The compounds are effectively administered orally, topically, or parenterally. A typical daily dosage contains from about 1 mg/$M^2$ to about 1,000 mg/$M^2$ of the compound of formula (I). The activity of representative compounds employed in the present invention has been demonstrated in standard screens commonly used by those of ordinary skill in the art to test for potential use of these compounds as solid and non-solid anti-tumor drugs. For example, these screens have been used to demonstrate the anti-tumor activity of commercially available cancer drugs such as the vinca alkaloids; see Miller, et al., J. Med. Chem., 20, No. 3, p 409 (1977); Sweeney, et al., Cancer Research, 38, p 2886 (1978) and Grindey, Cancer Cells, 2, p 163 (1990). The representative compounds of formula (I) employed in the present invention are cytotoxic as they inhibit the growth of rapidly dividing human leukemia cells (CCRF-CEM cell line). Table 1 below gives the results of testing several compounds representative of formula (I). In Table 1, Column 1 gives the representative compound and Column 2 gives the $IC_{50}$ (concentration giving 50 percent growth inhibition) in μg/ml. A compound having an $IC_{50}$ less than 20 μg/ml is considered to be active.

Table 1

| Compound | $IC_{50}$ (μg/ml) |
|---|---|
| (a) 2-deoxy-2,2-difluoro-D-ribofuranosyl-1-(2-amino-6-chloropurine) (Example 1); | 0.007 |
| (b) 2-deoxy-2,2-difluoro-D-ribofuranosyl-1-(2-amino-6-methoxypurine) (Example 2); | 0.037 |
| (c) 2-deoxy-2,2-difluoro-D-ribofuranosyl-1-(2-amino-6-ethoxypurine) (Example 3); | 0.2 |
| (d) 2-deoxy-2,2-difluoro-D-ribofuranosyl-1-(2-amino-6-dimethylaminopurine) (Example 4); | 4.6 |
| (e) 2-deoxy-2,2-difluoro-D-ribofuranosyl-1-(2-amino-6-methylaminopurine) (Example 5); | 0.054 |
| (f) 2-deoxy-2,2-difluoro-D-ribofuranosyl-1-(2-amino-6-iodopurine) (Example 6); | 0.093 |
| (g) 2-deoxy-2,2-difluoro-D-ribofuranosyl-1-(2-amino-6-cyanopurine) (Example 7); | 2.5 |
| (h) 2-deoxy-2,2-difluoro-D-ribofuranosyl-1-(2-amino-6-thiopurine); | 4.3 |
| (i) 2-deoxy-2,2-difluoro-D-ribofuranosyl-1-(2-hydroxyadenosine) (Example 9); | 0.7 |
| (j) 2-deoxy-2,2-difluoro-D-ribofuranosyl-1-(2-fluoroadenosine) (Example 10); | 0.094 |
| (k) 2-deoxy-2,2-difluoro-D-ribofuranosyl-1-purine (Example 11); | 1.3 |

(1) 2-deoxy-2,2-difluoro-D-ribofuranosyl-1-(2-amino-6-methylpurine) (Example 13); 2.6

To further demonstrate the ability of the compounds of formula (I) to treat susceptible neoplasms in mammals, the compounds of Examples 3 and 5 were tested in animals bearing a tumor system representative of HC1 human colon carcinoma. The HC1 human colon carcinoma xenograft was obtained in 1987 from Drs. Peter and Janet Houghton, St. Jude Children's Hospital, Memphis, TN. The tumor is a moderately well-differentiated adenocarcinoma of the ascending colon [British Journal of Cancer, 37, p 213-223 (1987)]. The first passage tumor was stored in liquid nitrogen using standard techniques. The tumors were maintained in serial passage Nu/Nu CD-1 mice (Charles River Laboratories, Portage, MI.).

The study testing the efficacy of these compounds against HC1 human colon carcinoma was initiated by removing a tumor from passage animals and mincing it into 1 to 3 mm square fragments using sterile techniques. Tumor pieces were checked for sterility using both an Antibiotic Medium 1 and Brain Heart Infusion (Difco; Detroit, MI). Tumor pieces were implanted subcutaneously in the auxilary region of recipient mice by trocar. Drug therapy on the appropriate schedule was initiated 14 days after tumor implantation. Drugs were dissolved in Emulphor for all experiments. All animals were weighed at the beginning and end of drug treatment. Food and water were provided ad libitum. On day 14 after the initiation of therapy, two dimensional measurements (width and length) of all tumors were taken using electronic calipers interfaced to a computer. Tumor weights were calculated from these measurements using the following formula:

$$\text{Tumor Weight (mg)} = \text{Tumor Length (mm)} \times \text{Tumor Width (mm)}^2/2$$

For all data, the tumor weight was rounded to the nearest tenth of a gram for analysis. No group is included in the analysis for therapeutic activity in which deaths attributable to drug toxicity exceeded 20 percent of the treated group. Compounds are considered active if more than 60 percent inhibition of tumor growth, as determined by tumor weight, is achieved at maximally tolerated doses.

In Table 2 which follows, Column 1 gives the example number of the compound tested; Column 2, the dose level; Column 3, the route administered; Column 4, the dosage schedule; Column 5, the percent inhibition of the tumor; and Column 6; the toxic deaths observed prior to completion of the study.

Table 2

| Example No. of Compound Tested | Dose Level mg/kg | Route of Administration | Dosage Schedule | Percent Inhibition of Tumor | Toxic Deaths |
|---|---|---|---|---|---|
| 3 | 100 | Intra-Peritoneal 0.5 ml | Daily X 10 | 98 | 0/10 |
| 3 | 50 | Intra-Peritoneal 0.5 ml | Daily X 10 | 97 | 0/10 |
| 3 | 25 | Intra-Peritoneal 0.5 ml | Daily X 10 | 98 | 0/10 |
| 5 | 80 | Intra-Peritoneal 0.5 ml | 1,3,5,7,9; 14 day delay | 95 | 0/8 |
| 5 | 40 | Intra-Peritoneal 0.5 ml | 1,3,5,7 9; 14 day delay | 94 | 1/8 |
| 5 | 20 | Intra-Peritoneal 0.5 ml | 1,3,5,7 9; 14 day delay | 93 | 0/8 |
| 5 | 10 | Intra-Peritoneal 0.5 ml | 1,3,5,7 9; 14 day delay | 92 | 0/8 |
| 5 | 05 | Intra-Peritoneal 0.5 ml | 1,3,5,7 9; 14 day delay | 92 | 0/8 |

In yet another aspect, the invention provides a method of treating viral infections in mammals comprising administering to a mammal in need of such treatment an anti-virally effective amount of a compound of formula (I).

The compounds of formula (I) and their pharmaceutically acceptable salts are effective for the treatment of viral infections in the manner usually employed in the treatment of such pathologies and can be used to treat or prevent infections caused by a wide range of viruses; and more particularly infections caused by viruses of the Herpes genus. Typical viruses against which the compounds of formula (I) can be used include all A and B strains of influenza, para-influenza, respiratory syncytial viruses, various Echo and vaccinia viruses, measles, Semliki Forest and retroviruses, Hepatitis B Virus, Human Cytomegalovirus and Human Immunodeficiency virus, the eitologic agent for Acquired Immune Deficiency Syndrome (AIDS).

The compounds of formula (I) are used for treating viral infections in the usual manner for treating such pathologies. The anti-viral effect of the compounds of formula (I) has been shown by proven in vitro test described below for Human Cytomegalovirus and Hepatitis B Virus carried out using representative compounds of formula (I).

Human Cytomegalovirus Test

WI-38 cells (American Type Culture Collection; CCL-35) were seeded into 24-well plates in MEM media (GIBCO) supplemented with 10% fetal bovine serum and penicillin/streptomycin. The plates wre incbated at 37°C, in 5 percent carbon dioxide in a humidified incubator for 3 days. Human Cytomegalovirus (HCMV) strain AD169 (American Type Culture Collection) was added to each well (40 plaque forming unites in 100 μl of media) and the virus was allowed to adsorb to the cells for 2 hours at room temperature. The compound of Example 5 was dissolved in 100 percent dimethylsulfoxide at a concentration of 20 mgs/ml and diluted with media to yield final concentrations of 50, 25, 12.5, 6.25, 3.13, 1.6, 0.8, 0.4, 0.2, and 0.1 μg/ml in one set of experiments and 32, 10, 3.2, 1, and 0.32 μg/ml in another set of experiments. Each concentration was done in duplicate. In all experiments, Cytovene (DHPG) was used as a positive control.

The infected cells were incubated for 10 days at 37°C, 5 percent carbon dioxide, in an humidified incubator without changes of media. After incubation, the cells were fixed for at least 15 minutes with 10 percent formalin and rinsed with water. After rinsing, the cells were stained for at least 15 minutes with crystal violet and allowed to dry. HCMV induced plaques were counted with the aid of an iverted microscope. The number of viral plaques appearing in cells treated with each concentration of the Example 5 compound was compared to the number

of viral plaques in cells left untreated ("no-drug control") and the percent inhibition of viral plaque formation was calculated. The concentration of the Example 5 compound required to inhibit viral plaque formation by 50 percent was determined using linear regression analysis. The results of these evaluations are reported below in Table 3.

Table 3

Percent Plaque Inhibition at Specified µg/ml conc.

| Compound | 50 | 25 | 12.5 | 6.25 | 3.12 | 1.6 | 0.8 | 0.4 | 0.2 |
|---|---|---|---|---|---|---|---|---|---|
| Example 5 | ST | 89.6 | 81 | 86.2 | 62 | 41.4 | 38 | 17.2 | 24 |
| Cytovene | 100 | 100 | 100 | 98 | 91 | 65.5 | 40 | 31 | 31 |

ST = SLIGHTLY TOXIC

Hepatitis B Virus Test

Plasmid Construction

Plasmids were constructed using standard recombinant DNA techniques; see Sambrook, J., *et al.,* Molecular Cloning: A Laboratory Manual, 2nd Edition (1989), Cold Spring Harbor Press, Cold Spring Harbor, NY. Plasmid pTHBV contains a head-to-tail dimer of the human Hepatitis B Virus (HBV) genome, subtype adw, clone into the EcoR I site of pUC9. Plasmid resistance under control of the SV40 early promotor with Bam HI from LTR-CAT-neo and subcloned into the Sal I site of pTHBV after a two base fill-in reaction with the Klenow fragment of E. coli DNA polymerase I.

Cells and Transfections

HepG2 cells (human hepatoblastoma, ATCC HB 8065) were routinely passaged in growth medium consisting of EARLE'S minimal essential media (Grand Island Biological Company (GIBCO) Bethesda Research Laboratories (BRL), Life Technologies, Inc., Gathersburg, MD.) supplemented with sodium pyruvate, 10 percent fetal bovine serum, 100 units/ml penicillin, and 100 µg/ml streptomycin. Hep10A cells were derived from HepG2 cells. Briefly, HepG2 cells were seeded into 60 mm culture dishes in growth medium, and when approximately 50 percent confluent, were transfected with pTHBVneo using the calcium phosphate precipitation method described by Graham, F.L. and Van der Eb in Virology, 52, p 456-467 (1973). After four hours of post-transfection, the cells were shocked with 10 percent dimethylsulfoxide and 20 percent glucose for 5 minutes, after which they were allowed to recover for 24 horus. The transfected cells were passaged and reseeded in the presence of growth medium containing 800 µg/ml geneticin. The cells were monitored for the appearance of geneticin resistant cells. A genecitin resistant cell line was established, and routinely passaged in growth medium containing 400 µg/ml geneticin. This cell line will be referred to as Hep10A.

Drug Treatments and Southern Blot Analysis

Hep10A cells were seeded into 96-well microtiter plates at a density of approximately 50,000 cells per well, allowed to grow to confluence, and maintained at confluence for 2 to 4 days to allow Hepatitus B Virus derivative HBV DNA levels to stabilize; see Sells, M.A., *et al.,* J. Virol., 62, p 2836-2844 (1987) and Korba, B.E., *et al.,* Antiviral Res., 15, p 217-228 (1991). At this time, cells were treated with 2'-deoxy-2'-fluoroarabino-5-iodouridine (FIAU) and the compound of Example 5 for 10 days, with daily changes of media containing fresh drug. FIAU was used as a positive control. On day 10, culture supernatant samples were taken and stored at 4°C until needed. PCR analysis was performed on supernatant samples using appropriate primers to amplify a 477 base pair fragment from the "a" determinant of the surface antigen gene of HBV subtype adw. Following electrophoresis through agarose gels, the DNA was transferred to nylon membranes (Optiblot™, IBI, New Haven, CT) by capillary action (Southern, 1975) using 1X saline sodium citrate (SSC) (1X SSC=0.15 M NaCl, 0.015

M sodium citrate). After baking *in vacuo* for 30 minutes, the blots were hybridized for 15 to 18 hours to $1x10^6$ cpm/ml of $^{32}$p-labelled by the method of random priming (Feinberg and Vogelstein, 1983). Hybridization was carried out at 42°C using 50 percent N,N-dimethylformamide, 6X saline sodium phosphate ethylenediamine tetraacetic acid (SSPE), 1.0 percent sodium dodyl sulfate (SDS), and 50 µg/ml salmon sperm DNA (1X SSPE=0.18 M NaCl; 10 nM $NaH_2PO_4$, pH 7.4; 1 mmol ethylene diamine tetraacetic acid (EDTA)). The hybridized blots were washed as follows: twice in 2X SSC/0.1 percent SDS at room temperature for 1 hour, once in 0.1X SSC/0.1 percent SDS at room temperature for 30 minutes, and once in 0.1x SSC/0.1 percent supplemented with SDS at 65°C for 15 minutes. The filters were exposed to x-ray film and quantitated by densitometry. The results of these evaluations are reported below in Table 4.

Table 4

| Percent Plaque Inhibition at Specified µg/ml conc. | | | | |
|---|---|---|---|---|
| Compound | 0.1 | 1.0 | 10.0 | 100.0 |
| Example 5 | 56.9 | 81.9 | 84.6 | 83.9 |
| FIAU | 10.8 | 35.5 | 65.3 | 81.7 |

From the above data, the concentration of FIAU and the Example 5 compound required to inhibit HBV DNA replication by 50 percent was 5.4 µg/ml and <0.1 µg/ml, respectively.

## Claims

1. A compound of the formula

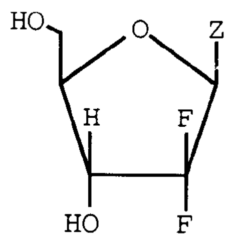

(I);

wherein Z is selected from the group consisting of:

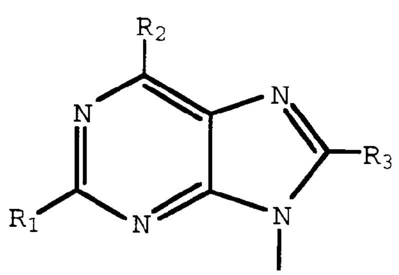

,

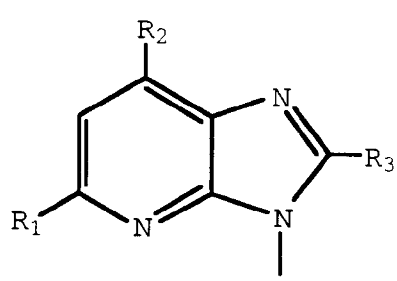

,

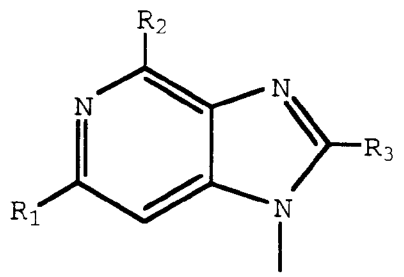 , 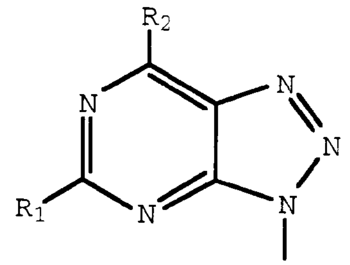 ,

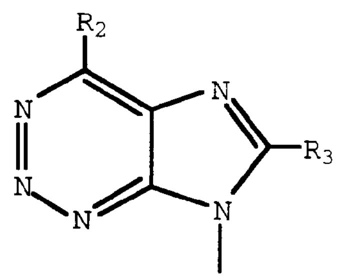 and 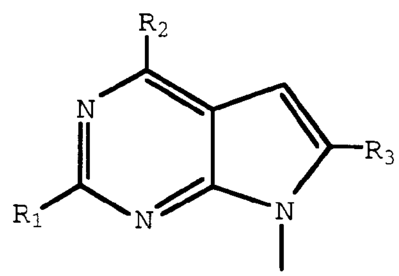 ;

wherein $R_1$ and $R_2$ are independently selected from the group consisting of alkyl, alkoxy, amine, halo, hydrogen, hydroxy, cyano, thio, thioalkyl, hydrazide, carboxamide, thioamide, sulfonamide, sulfinamide, alkylamine, thioamine, hydroxyamine, NH(alkyl), $N(alkyl)_2$, O(aryl), O(substituted aryl), N(aryl), N(substituted aryl); and $R_3$ is selected from the group consisting of hydrogen, hydroxy, amine, NH(alkyl), halo, alkoxy, thioalkyl; and pharmaceutically acceptable salts thereof.

**2.** A compound according to Claim 1 selected from the group consisting of:
(a) 2-deoxy-2,2-difluoro-D-ribofuranosyl-1-β-(2-amino-6-chloropurine);
(b) 2-deoxy-2,2-difluoro-D-ribofuranosyl-1-β-(2-amino-6 methoxypurine);
(c) 2-deoxy-2,2-difluoro-D-ribofuranosyl-1-β-(2-amino-6-ethoxypurine);
(d) 2-deoxy-2,2-difluoro-D-ribofuranosyl-1-β-(2-amino-6-dimethylaminopurine);
(e) 2-deoxy-2,2-difluoro-D-ribofuranosyl-1-β-(2-amino-6-methylaminopurine);
(f) 2-deoxy-2,2-difluoro-D-ribofuranosyl-1-β-(2-amino-6-iodopurine);
(g) 2-deoxy-2,2-difluoro-D-ribofuranosyl-1-β-(2-amino-6-cyanopurine);
(h) 2-deoxy-2,2-difluoro-D-ribofuranosyl-1-β-(2-amino-6-thiopurine)
(i) 2-deoxy-2,2-difluoro-D-ribofuranosyl-1-β-(2-amino-6-carboxamidopurine);
(j) 2-deoxy-2,2-difluoro-D-ribofuranosyl-1-β-(2-hydroxyadenosine);
(k) 2-deoxy-2,2-difluoro-D-ribofuranosyl-1-β-(2-fluoroadenosine);
(l) 2-deoxy-2,2-difluoro-D-ribofuranosyl-1-β-purine;
(m) 2-deoxy-2,2-difluoro-D-ribofuranosyl-1-β-(2-amino-6-methylpurine);
(n) 2-deoxy-2,2-difluoro-D-ribofuranosyl-1-β-(6-chloro-7-deazapurine);
(o) 2-deoxy-2,2-difluoro-D-ribofuranosyl-1-β-(2,6-dichloro-3-deazapurine);
(p) 2-deoxy-2,2-difluoro-D-ribofuranosyl-1-β-(6-amino-3-deazapurine);
(q) 2-deoxy-2,2-difluoro-D-ribofuranosyl-1-β-(2,6-chloropurine);
and pharmaceutically acceptable salts thereof.

**3.** A pharmaceutical composition useful for treating susceptible neoplasms and viral infections in mammals comprising a pharmaceutically effective amount of a suitable pharmaceutically acceptable carrier, diluent or excipient and a compound of Claim 1.

**4.** A pharmaceutical composition according to Claim 3 wherein the compound of Claim 1 is selected from the group consisting of:
(a) 2-deoxy-2,2-difluoro-D-ribofuranosyl-1-β-(2-amino-6-chloropurine);
(b) 2-deoxy-2,2-difluoro-D-ribofuranosyl-1-β-(2-amino-6 methoxypurine);
(c) 2-deoxy-2,2-difluoro-D-ribofuranosyl-1-β-(2-amino-6-ethoxypurine);

(d) 2-deoxy-2,2-difluoro-D-ribofuranosyl-1-β-(2-amino-6-dimethylaminopurine);
(e) 2-deoxy-2,2-difluoro-D-ribofuranosyl-1-β-(2-amino-6-methylaminopurine);
(f) 2-deoxy-2,2-difluoro-D-ribofuranosyl-1-β-(2-amino-6-iodopurine);
(g) 2-deoxy-2,2-difluoro-D-ribofuranosyl-1-β-(2-amino-6-cyanopurine);
(h) 2-deoxy-2,2-difluoro-D-ribofuranosyl-1-β-(2-amino-6-thiopurine)
(i) 2-deoxy-2,2-difluoro-D-ribofuranosyl-1-β-(2-amino-6-carboxamidopurine);
(j) 2-deoxy-2,2-difluoro-D-ribofuranosyl-1-β-(2-hydroxyadenosine);
(k) 2-deoxy-2,2-difluoro-D-ribofuranosyl-1-β-(2-fluoroadenosine);
(l) 2-deoxy-2,2-difluoro-D-ribofuranosyl-1-β-purine;
(m) 2-deoxy-2,2-difluoro-D-ribofuranosyl-1-β-(2-amino-6-methylpurine);
(n) 2-deoxy-2,2-difluoro-D-ribofuranosyl-1-β-(6-chloro-7-deazapurine);
(o) 2-deoxy-2,2-difluoro-D-ribofuranosyl-1-β-(2,6-dichloro-3-deazapurine);
(p) 2-deoxy-2,2-difluoro-D-ribofuranosyl-1-β-(6-amino-3-deazapurine);
(q) 2-deoxy-2,2-difluoro-D-ribofuranosyl-1-β-(2,6-chloropurine).

**5.** A method of treating viral infections in a mammal suffering from said condition comprising administering to a mammal in need of such treatment a pharmaceutically effective amount of a compound of Claim 1.

**6.** A method according to Claim 5 wherein the compound of Claim 1 is selected from the group consisting of:
(a) 2-deoxy-2,2-difluoro-D-ribofuranosyl-1-β-(2-amino-6-chloropurine);
(b) 2-deoxy-2,2-difluoro-D-ribofuranosyl-1-β-(2-amino-6 methoxypurine);
(c) 2-deoxy-2,2-difluoro-D-ribofuranosyl-1-β-(2-amino-6-ethoxypurine);
(d) 2-deoxy-2,2-difluoro-D-ribofuranosyl-1-β-(2-amino-6-dimethylaminopurine);
(e) 2-deoxy-2,2-difluoro-D-ribofuranosyl-1-β-(2-amino-6-methylaminopurine);
(f) 2-deoxy-2,2-difluoro-D-ribofuranosyl-1-β-(2-amino-6-iodopurine);
(g) 2-deoxy-2,2-difluoro-D-ribofuranosyl-1-β-(2-amino-6-cyanopurine);
(h) 2-deoxy-2,2-difluoro-D-ribofuranosyl-1-β-(2-amino-6-thiopurine)
(i) 2-deoxy-2,2-difluoro-D-ribofuranosyl-1-β-(2-amino-6-carboxamidopurine);
(j) 2-deoxy-2,2-difluoro-D-ribofuranosyl-1-β-(2-hydroxyadenosine);
(k) 2-deoxy-2,2-difluoro-D-ribofuranosyl-1-β-(2-fluoroadenosine);
(l) 2-deoxy-2,2-difluoro-D-ribofuranosyl-1-β-purine;
(m) 2-deoxy-2,2-difluoro-D-ribofuranosyl-1-β-(2-amino-6-methylpurine);
(n) 2-deoxy-2,2-difluoro-D-ribofuranosyl-1-β-(6-chloro-7-deazapurine);
(o) 2-deoxy-2,2-difluoro-D-ribofuranosyl-1-β-(2,6-dichloro-3-deazapurine);
(p) 2-deoxy-2,2-difluoro-D-ribofuranosyl-1-β-(6-amino-3-deazapurine);
(q) 2-deoxy-2,2-difluoro-D-ribofuranosyl-1-β-(2,6-chloropurine);
and pharmaceutically acceptable salts thereof.

**7.** A method of treating susceptible neoplasms in a mammal suffering from said condition comprising administering to a mammal in need of such treatment a pharmaceutically effective amount of a compound of Claim 1.

**8.** A method according to Claim 7 wherein the compound of Claim 1 is selected from the group consisting of:
(a) 2-deoxy-2,2-difluoro-D-ribofuranosyl-1-β-(2-amino-6-chloropurine);
(b) 2-deoxy-2,2-difluoro-D-ribofuranosyl-1-β-(2-amino-6 methoxypurine);
(c) 2-deoxy-2,2-difluoro-D-ribofuranosyl-1-β-(2-amino-6-ethoxypurine);
(d) 2-deoxy-2,2-difluoro-D-ribofuranosyl-1-β-(2-amino-6-dimethylaminopurine);
(e) 2-deoxy-2,2-difluoro-D-ribofuranosyl-1-β-(2-amino-6-methylamino purine);
(f) 2-deoxy-2,2-difluoro-D-ribofuranosyl-1-β-(2-amino-6-iodopurine);
(g) 2-deoxy-2,2-difluoro-D-ribofuranosyl-1-β-(2-amino-6-cyanopurine);
(h) 2-deoxy-2,2-difluoro-D-ribofuranosyl-1-β-(2-amino-6-thiopurine)
(i) 2-deoxy-2,2-difluoro-D-ribofuranosyl-1-β-(2-amino-6-carboxamidopurine);
(j) 2-deoxy-2,2-difluoro-D-ribofuranosyl-1-β-(2-hydroxyadenosine);
(k) 2-deoxy-2,2-difluoro-D-ribofuranosyl-1-β-(2-fluoroadenosine);
(l) 2-deoxy-2,2-difluoro-D-ribofuranosyl-1-β-purine;
(m) 2-deoxy-2,2-difluoro-D-ribofuranosyl-1-β-(2-amino-6- methylpurine);

(n) 2-deoxy-2,2-difluoro-D-ribofuranosyl-1-β-(6-chloro-7-deazapurine);
(o) 2-deoxy-2,2-difluoro-D-ribofuranosyl-1-β-(2,6-dichloro-3-deazapurine);
(p) 2-deoxy-2,2-difluoro-D-ribofuranosyl-1-β-(6-amino-3-deazapurine);
(q) 2-deoxy-2,2-difluoro-D-ribofuranosyl-1-β-(2,6-chloropurine);
and pharmaceutically acceptable salts thereof.

**9.** An intermediate of the formula

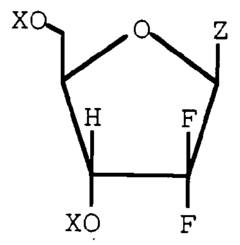

(II);

wherein X is a hydroxy protecting group and Z is a purine nucleobase selected from the group consisting of:

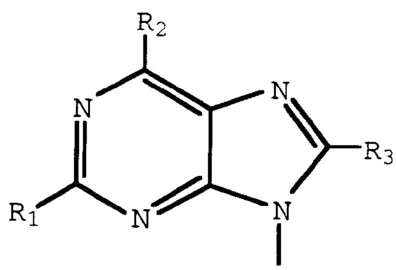
,
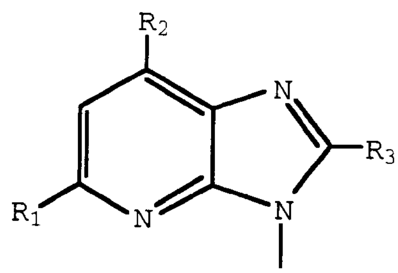
,

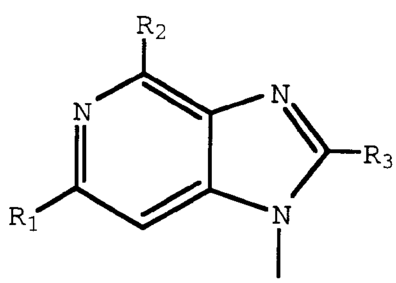
,
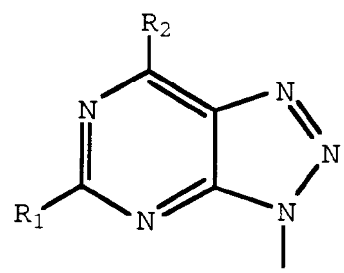
,

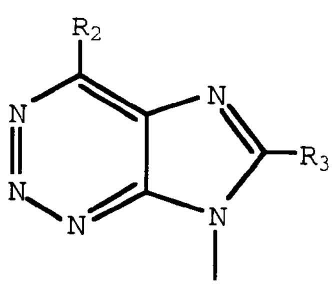
and
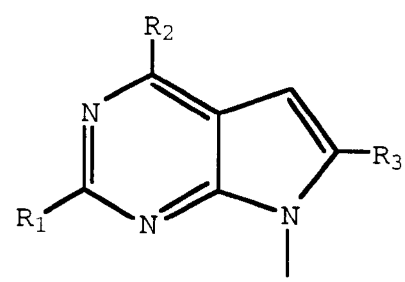
;

wherein $R_1$ and $R_2$ are independently selected from the group consisting of alkyl, alkoxy, amine, halo, hydrogen, hydroxy, cyano, thio, thioalkyl hydrazide, carboxamide, thioamide, sulfonamide, sulfinamide,

aklylamine, thioamine, hydroxyamine, NH(alkyl), $N(alkyl)_2$, O(aryl), O(substituted aryl), NH(aryl), N(substituted aryl); and $R_3$ is selected from the group consisting of hydrogen, hydroxy, amine, NH(alkyl), halo, alkoxy, thioalkyl; and pharmaceutically acceptable salts thereof.

**10.** An intermediate according to Claim 9 selected from the group consisting of
(a) 2-deoxy-2,2-difluoro-D-ribofuranosyl-3,5-dibenzoyl-1-β-(2-amino-6-chloropurine);
(b) 2-deoxy-2,2-difluoro-D-ribofuranosyl-3,5-dibenzoyl-1-β-2-amino-6-methoxypurine);
(c) 2-deoxy-2,2-difluoro-D-ribofuranosyl-3,5-dibenzoyl-1-β-(2-amino-6-ethoxypurine);
(d) 2-deoxy-2,2-difluoro-D-ribofuranosyl-3,5-dibenzoyl-1-β-(2-amino-6-dimethylaminopurine);
(e) 2-deoxy-2,2-difluoro-D-ribofuranosyl-3,5-dibenzoyl-1-β-(2-amino-6-methylaminopurine);
(f) 2-deoxy-2,2-difluoro-D-ribofuranosyl-3,5-dibenzoyl-1-β-(2-amino-6-iodopurine);
(g) 2-deoxy-2,2-difluoro-D-ribofuranosyl-3,5-dibenzoyl-1-β-(2-amino-6-cyanopurine);
(h) 2-deoxy-2,2-difluoro-D-ribofuranosyl-3,5-dibenzoyl-1-β-(2-amino-6-thiopurine)
(i) 2-deoxy-2,2-difluoro-D-ribofuranosyl-3,5-dibenzoyl-1-β-(2-amino-6-carboxamidopurine);
(j) 2-deoxy-2,2-difluoro-D-ribofuranosyl-3,5-dibenzoyl-1-β-(2-hydroxyadenosine);
(k) 2-deoxy-2,2-difluoro-D-ribofuranosyl-3,5-dibenzoyl-1-β-(2-fluoroadenosine);
(l) 2-deoxy-2,2-difluoro-D-ribofuranosyl-3,5-dibenzoyl-1-β-purine;
(m) 2-deoxy-2,2-difluoro-D-ribofuranosyl-3,5-dibenzoyl-1-β-(2-aminopurine);
(n) 2-deoxy-2,2-difluoro-D-ribofuranosyl-3,5-dibenzoyl-1-β-(2-amino-6-methylpurine);
(o) 2-deoxy-2,2-difluoro-D-ribofuranosyl-3,5-dibenzoyl-1-β-(2,6-chloro-1-deazapurine);
(p) 2-deoxy-2,2-difluoro-D-ribofuranosyl-3,5-dibenzoyl-1-β-(6-chloro-7-deazapurine);
(q) 2-deoxy-2,2-difluoro-D-ribofuranosyl-3,5-dibenzoyl-1-β-(dichloro-3-deazapurine);
(r) 2-deoxy-2,2-difluoro-D-ribofuranosyl-3,5-dibenzoyl-1-β-(6-amino-3-deazapurine); and
(s) 2-deoxy-2,2-difluoro-D-ribofuranosyl-3,5-dibenzoyl-1-β-(2,6-chloropurine); and pharmaceutically acceptable
salts thereof.